# EUROPEAN PATENT SPECIFICATION

(11) **EP 4 321 868 B1**
(45) Date of publication and mention of the grant of the patent: **24.06.2026**
(21) Application number: 22785022.9
(22) Date of filing: 08.04.2022
(51) Int. Cl.: G01N 33/68, G01N 33/574, A61K 39/00, A61P 35/00

(54) **METHOD FOR TREATING CANCER USING IMMUNE CHECKPOINT INHIBITOR**
VERFAHREN ZUR BEHANDLUNG VON KREBS MIT EINEM IMMUNCHECKPOINT-INHIBITOR
MÉTHODE DE TRAITEMENT DU CANCER À L'AIDE D'UN INHIBITEUR DE POINT DE CONTRÔLE IMMUNITAIRE

(30) Priority: 09.04.2021 US 202163172950 P; 12.04.2021 US 202163173550 P
(43) Date of publication of application: 14.02.2024
(73) Proprietor: Cytogen, Inc., Seoul 05854 (KR)
(72) Inventor: JEON, Byung Hee, Seongnam-si Gyeonggi-do 13611 (KR)
(74) Representative: Mewburn Ellis LLP
(86) International application number: PCT/KR2022/005130
(87) International publication number: WO 2022/216113

(56) References cited:
- WO-A1-2017/181073
- KR-A- 20180 048 215
- JENNIFER L. SCHEHR ET AL: "High Specificity in Circulating Tumor Cell Identification Is Required for Accurate Evaluation of Programmed Death-Ligand 1", PLOS ONE, vol. 11, no. 7, 26 July 2016 (2016-07-26), pages e0159397, XP055595664, DOI: 10.1371/journal.pone.0159397
- HE YUTONG, SHI JIN, SCHMIDT BERND, LIU QINGYI, SHI GAOFENG, XU XIAOLI, LIU CONGMIN, GAO ZHAOYU, GUO TIANTIAN, SHAN BAOEN: "Circulating Tumor Cells as a Biomarker to Assist Molecular Diagnosis for Early Stage Non-Small Cell Lung Cancer", CANCER MANAGEMENT AND RESEARCH, vol. Volume 12, pages 841 - 854, XP055976587, DOI: 10.2147/CMAR.S240773
- KLOTEN, LAMPIGNANO, KRAHN, SCHLANGE: "Circulating Tumor Cell PD-L1 Expression as Biomarker for Therapeutic Efficacy of Immune Checkpoint Inhibition in NSCLC", CELLS, vol. 8, no. 8, pages 809, XP055976586, DOI: 10.3390/cells8080809
- ZHANG TIAN, AGARWAL ANIKA, ALMQUIST R. GARLAND, RUNYAMBO DANIELLA, PARK SALLY, BRONSON ELIZABETH, BOOMINATHAN RENGASAMY, RAO CHAND: "Expression of immune checkpoints on circulating tumor cells in men with metastatic prostate cancer", BIOMARKER RESEARCH, vol. 9, no. 1, 1 December 2021 (2021-12-01), XP055976584, DOI: 10.1186/s40364-021-00267-y
- KIM, SEUNG IL: "Circulating Tumor Cells in Breast Cancer", JOURNAL OF BREAST DISEASE, vol. 1, no. 1, 19 November 2013 (2013-11-19), pages 2 - 7, XP009540330, ISSN: 2288-5560, DOI: 10.14449/jbd.2013.1.11

## Description

### [Technical Field]

The present invention relates to a method of treating cancer (not claimed as such but in the form of a further medical use, see independent claim 15) using an immune checkpoint inhibitor, and more particularly to a method of treating cancer by confirming expression of an immune checkpoint protein and a lymphoid or myeloid cell-specific protein in isolated circulating tumor cells isolated from the blood, selecting a patient in whom immune checkpoint protein expression is confirmed as positive and lymphoid or myeloid cell-specific protein expression is confirmed as positive as a patient to whom cancer immunotherapy drug is applicable, and administering an immune checkpoint inhibitor to the patient.

### [Background Art]

Cancer is one of the most devastating threats to human health. There are nearly 1.3 million new cancer cases each year in the United States and cancer is the second leading cause of death after heart disease, killing 1 in 4 people. Cancer is also expected to surpass cardiovascular disease as the number one cause of death within five years. Solid tumors are responsible for most of such deaths.

Although various methods have been attempted to treat cancer, the overall 5-year survival rate of any cancer has only improved by about 10% over the past 20 years because the clinical results and prognosis of patients do not always match the reported data. Therefore, it is possible to provide the most appropriate method of treating cancer patients with heterogeneous characteristics by predicting the drug response and prognosis of cancer patients in anticancer treatment in advance, thus avoiding unnecessary treatment-related toxicity and ultimately increasing the therapeutic effect.

In accordance with this need, thorough research into companion diagnostics, which is an approved diagnostic test capable of selecting an appropriate targeted therapy and treatment method based on results of systematic analysis of patient's individual factors, is ongoing. Companion diagnostics is capable of providing a clear clinical basis for prescription according to the doctor's diagnosis, and suggesting an appropriate treatment method to the patient, which not only increases the efficiency of cancer treatment, but also reduces the misuse and abuse of targeted therapy, thereby improving the financial soundness of the national health insurance. Currently, the companion diagnostics market is growing in the fields of treatment of breast cancer, lung cancer, large-bowel cancer, gastric cancer, melanoma, and the like, and in particular, the fields of breast cancer and lung cancer are expected to drive market growth. As new drug development costs of pharmaceutical companies are decreased and demand for targeted therapies is increased, the global companion diagnostics market is growing at a high growth rate every year.

Immunotherapy, on the other hand, uses the patient's own immune system to attack cancer regardless of origin thereof. The immune system is regulated by an enhanced network of checks and balances to attack foreign invaders such as bacteria and viruses. However, cancer may evade the immune system by expressing proteins that inhibit the immune system from attacking cancer cells, such as PD-L1 and PD-L2.

In particular, the interaction between tumor cells and T cells involves contact between the major histocompatibility complex (MHC) on the tumor cells and the T cell receptor (TCR) on the T cells. Upon contact between the MHC and the T cell receptor, the T cells are activated and the tumor cells are destroyed.

If tumor cells express PD-L1 as an immune checkpoint protein on the surface thereof, they may evade T cell immunosurveillance. PD-L1, when present, inhibits T cell immunosurveillance by binding to PD-1 expressed by T cells and blocking T cell activation.

Immune checkpoint inhibitors capable of blocking PD-L1/PD-1 interactions have been developed. Such drugs allow T cell immunosurveillance mechanisms to function normally again, so that tumor cells may be destroyed through a normal immune response in a subject. Blockade of CTLA-4 on T cells may have similar effects (Hodi et al., N. Engl. J. Med. Vol. 363, pp. 711-23, 2010).

The key to effective use of immune checkpoint inhibitors is to determine whether a particular subject suffering from cancer responds to the drug. If an antibody that binds to PD-L1 or PD-1 and acts as an immune checkpoint inhibitor were to be administered to a patient whose tumor cells do not express PD-L1, treatment would be ineffective. Until now, the response of these immune checkpoint inhibitors is merely 20-30% (Herbst RS, et al. The New England Journal of Medicine, Vol. 383(14), pp. 1328-39, 2020).

For measurement of the expression level of PD-L1, a method of calculating the tumor proportion score (TPS) of PD-L1 in tissue cells of patients is currently recommended. For example, in order for pembrolizumab (Keytruda) and nivolumab (Opdivo), which are antibodies that specifically act on PD-1, to work effectively, the expression level of PD-L1 in a patient's tissue test has to be detected at a certain percentage or higher. To be covered by health insurance benefits, PD-L1 has to be detected at 10% or more in Opdivo or 50% or more in Keytruda, and atezolizumab (Tecentriq) requires detection of PD-L1 expression at 5% or more in both tumor cells and tumor-infiltrating immune cells.

However, obtaining cancer cells via tumor biopsy to investigate PD-L1 expression has serious drawbacks, such as pain and discomfort to patients, non-investigated isolated tumor region abnormalities, and potential for protein expression profiles to change over time in a tumor microenvironment. Re-biopsy is also invasive and entails other complicated problems. Moreover, since patient discrimination criteria for immune checkpoint inhibitors are different from each other, it is very difficult to select the most effective treatment for patients.

With the goal of overcoming such disadvantages, liquid biopsy including circulating tumor cells (CTCs), circulating cell-free DNA (cfDNA), and exosomes is recently receiving attention as a new solution (Rijavec E, et al., Cancers. Vol. 12(1):17, 2020). Blood-based biopsy is superior to tissue biopsy in that cells delaminated or otherwise isolated by the tumor may be tracked sequentially in real time.

Blood samples are more easily obtainable and may be collected more frequently from patients. Additionally, changes in the protein expression profile may be monitored over time. Circulating tumor cells (CTCs) are one of the cancer-associated cell types that may be readily isolated from peripheral blood and used as a substitute for tumor cells obtained from tissue biopsy. CTCs are tumor cells isolated from solid tumors into the bloodstream. CTCs may be found in the blood of patients with carcinoma, sarcoma, neuroblastoma, or melanoma. The identification of additional cell types that may be obtained from blood-based biopsy is regarded as important to further develop the use of this technology to identify cancer patients who benefit from treatment with immune checkpoint inhibitors.

Against this technical background, the present inventors have made great efforts to develop a CTC-based cancer treatment method, and thus ascertained that expression of an immune checkpoint protein and expression of a lymphoid or immune cell-specific protein are simultaneously confirmed, and when an immune checkpoint inhibitor is administered to a patient in whom immune checkpoint protein expression is positive and lymphoid or immune cell-specific protein expression is positive, the same level of cancer treatment effect as a tissue test may be exhibited even without performing a tissue test, thus culminating in the present invention.

### [Disclosure]

It is an object of the present invention to provide a method of selecting a patient for application of an immune checkpoint inhibitor as defined by the claims.

It is another object of the present invention to provide an immune checkpoint inhibitor for use in a method of treating cancer as defined by the claims.

Provided herein, but not as claimed invention, is a method of determining susceptibility to an immune checkpoint inhibitor.

Provided herein, but not as claimed invention,I s a method of selecting cancer immunotherapy for a cancer patient.

In order to accomplish the above objects, the present invention provides a method of selecting a patient for application of cancer immunotherapy drug, including (a) confirming expression of an immune checkpoint protein and a lymphoid or myeloid-specific protein in isolated circulating tumor cells and (b) classifying a patient in whom immune checkpoint protein expression is confirmed as positive and lymphoid or myeloid-specific protein expression is confirmed as positive as a patient to whom cancer immunotherapy drug is applicable.

In addition, the present invention provides an immune checkpoint inhibitor for use in a method of treating cancer, the method including:
(a) confirming expression of an immune checkpoint protein and a lymphoid or myeloid-specific protein in isolated circulating tumor cells and (b) administering an immune checkpoint inhibitor to a patient in whom immune checkpoint protein expression is confirmed as positive and lymphoid or myeloid-specific protein expression is confirmed as positive.

Provided herein, but not as claimed invention, is a method of determining susceptibility to an immune checkpoint inhibitor, including:
(a) confirming expression of an immune checkpoint protein and a lymphoid or myeloid-specific protein in isolated circulating tumor cells and (b) determining that there is susceptibility to an immune checkpoint inhibitor when immune checkpoint protein expression is confirmed as positive and lymphoid or myeloid-specific protein expression is confirmed as positive.

Provided herein, but not as claimed invention, is a method of selecting cancer immunotherapy for a cancer patient, including:
(a) confirming expression of an immune checkpoint protein and a lymphoid or myeloid-specific protein in isolated circulating tumor cells and (b) selecting an immune checkpoint inhibitor as therapy when immune checkpoint protein expression is confirmed as positive and lymphoid or myeloid-specific protein expression is confirmed as positive.

Disclosed herein, but not as claimed invention, is:
a pharmaceutical composition for cancer treatment including an immune checkpoint inhibitor for treating a subject with positive immune checkpoint protein expression and positive lymphoid or myeloid-specific protein expression.

Provided herein, but not as claimed invention, is the use of an immune checkpoint inhibitor for the manufacture of a medicament for treating a subject with positive immune checkpoint protein expression and positive lymphoid or myeloid-specific protein expression.

### [Description of Drawings]

FIG. 1 shows fluorescence images of circulating tumor cells expressing EpCAM/Vimentin, PD-L1, and CD18 proteins according to an embodiment of the present invention;
FIG. 2 shows images obtained by measuring fluorescence intensities of EpCAM/Vimentin, PD-L1, and CD18 proteins in circulating tumor cells according to an embodiment of the present invention;
FIG. 3 shows fluorescence images (A) confirming the expression of CD16 and CD18 in blood samples of patients and graphs (B) quantifying the same according to an embodiment of the present invention; and
FIG. 4 is a heatmap for the cell number and fluorescence intensity of EpCAM/Vimentin, PD-L1, and CD18 according to an embodiment of the present invention.

### [Mode for Invention]

Unless otherwise defined, all technical and scientific terms used herein have the same meanings as typically understood by those skilled in the art to which the present invention belongs. In general, the nomenclature used herein and experimental methods described below are well known in the art and are typical.

Terms such as first, second, A, B, and the like may be used to describe various components, but the components are not limited by the above terms, and these terms are used only for the purpose of distinguishing one component from another component. For example, the first component may be referred to as the second component, and similarly, the second component may also be referred to as the first component, without departing from the scope of the technology to be described below. The term "and/or" includes a combination of a plurality of related listed items or any item of a plurality of related listed items.

For the terms used herein, the singular forms are intended to include the plural forms as well, unless the context clearly indicates otherwise, and terms such as "comprise", "include", and the like specify the presence of stated features, numbers, steps, operations, elements, components, or combinations thereof, but do not preclude the presence or addition of one or more other features, numbers, steps, operations, elements, components, or combinations thereof.

In addition, in performing a method or operation, individual steps constituting the method may occur in a different order from the specified order unless a specific order is clearly described in context. Specifically, individual steps may occur in the same order as specified, may be performed substantially simultaneously, or may be performed in reverse order.

In the present invention, the term "antibody" is used in the broadest sense, and examples thereof include, but are not limited to, monoclonal antibodies, polyclonal antibodies, multispecific antibodies (e.g. bispecific antibodies), and antibody fragments, so long as they exhibit desired antigen-binding activity, and include various antibody structures.

As used herein, the term "biomarker" refers to an indicator that may be detected in a sample. A biomarker may serve as a predictive, diagnostic, and/or prognostic indicator of a disease or disorder (e.g. cancer) characterized by specific molecular, pathological, histological, and/or clinical features.

As used herein, the terms "cancer" and "cancerous" refer to or describe a physiological condition in mammals that is typically characterized by unregulated cell growth. Examples of cancer include, but are not limited to, carcinoma, lymphoma, blastoma, sarcoma, leukemia, and lymphoid malignancies. More specific examples of cancer include lung cancer including small cell lung cancer, non-small cell lung cancer, lung adenocarcinoma, and squamous cell carcinoma of the lung; bladder cancer (e.g. urinary bladder cancer (UBC), muscle invasive bladder cancer (MIBC), and BCG-unresponsive nonmuscle invasive bladder cancer (NMIBC)); kidney or renal cancer (e.g. renal cell carcinoma (RCC)); urinary tract cancer; breast cancer (e.g. HER2+ breast cancer and triple-negative breast cancer (TNBC) with negative expression of estrogen receptor (ER-), progesterone receptor (PR-), and HER2 (HER2-)); prostate cancer such as castrate-resistant prostate cancer (CRPC); peritoneal cancer; hepatocellular carcinoma; stomach or gastric cancer, including gastrointestinal cancer and gastrointestinal stromal cancer; pancreatic cancer; glioblastoma; cervical cancer; ovarian cancer; liver cancer; liver tumor; colon cancer; rectal cancer; colorectal cancer; endometrial or uterine carcinoma; salivary gland carcinoma; prostate cancer; vulvar cancer; thyroid cancer; liver carcinoma; anal carcinoma; penile carcinoma; melanoma, including superficial spreading melanoma, lentigo maligna melanoma, acral lentiginous melanoma, and nodular melanoma; multiple myeloma and B-cell lymphoma (including low-grade/follicular non-Hodgkin's lymphoma (NHL), small lymphocytic (SL) NHL, intermediate-grade/follicular NHL, intermediate-grade diffuse NHL, high-grade immunoblastic NHL, high-grade lymphoblastic NHL, high-grade small noncleaved cell NHL, bulky disease NHL, mantle cell lymphoma, AIDS-related lymphoma, and Waldenstrom macroglobulinemia); chronic lymphocytic leukemia (CLL); acute lymphoblastic leukemia (ALL); acute myelogenous leukemia (AML); hairy cell leukemia; chronic myeloblastic leukemia (CML); post-transplant lymphoproliferative disorder (PTLD); and myelodysplastic syndrome (MDS), as well as abnormal vascular proliferation associated with nevus, edema (e.g. associated with brain tumors), Meigs syndrome, brain cancer, head and neck cancer, and related metastases.

As used herein, the terms "treat", "treating", and "treatment" have general and customary meanings, which include at least one of completely or partially eliminating a tumor or cancer from a subject, reducing the size of a tumor in a subject, killing tumor or cancer cells in a subject, or ameliorating symptoms of cancer or a tumor in a subject. By treatment is meant elimination, reduction, killing, or amelioration by about 1% to about 100% compared to subjects not receiving the immune checkpoint inhibitor. Preferably, elimination, reduction, killing, or amelioration is about 100%, about 99%, about 98%, about 97%, about 96%, about 95%, about 90%, about 80%, about 70%, about 60% %, about 50%, about 40%, about 30%, about 20%, about 10%, about 5%, or about 1%. The treatment result may be permanent, or may persist over a period of days (e.g. 1, 2, 3, 4, 5, 6, or 7 days), weeks (e.g. 1, 2, 3, or 4 weeks), months (e.g. 1, 2, 3, 4, 5, 6 months or longer), or years (e.g. 1, 2, 3, 4, 5, 6 years or longer).

As used herein, the terms "inhibit", "inhibiting", and "inhibition" have general and customary meanings, which include at least one of hindering the establishment of cancer or a tumor, the development of cancer or a tumor, and the growth and metastasis of cancer or a tumor, or delaying, blocking, stopping, or restraining the progress thereof. By inhibition is meant hindering by about 1% to about 100% compared to subjects not receiving the immune checkpoint inhibitor. Preferably, hindering is about 100%, about 99%, about 98%, about 97%, about 96%, about 95%, about 90%, about 80%, about 70%, about 60%, about 50%, about 40%, about 30%, about 20%, about 10%, about 5%, or about 1%. The inhibition method may be performed in the subject before, concurrently with, or after the onset of clinical symptoms of cancer or a tumor. Thus, the subject may be a subject suffering from cancer or a tumor, or a subject only prone to develop cancer or a tumor. The inhibition result may be permanent, or may persist over a period of days (e.g. 1, 2, 3, 4, 5, 6, or 7 days), weeks (e.g. 1, 2, 3, or 4 weeks), months (e.g. 1, 2, 3, 4, 5, 6 months or longer), or years (e.g. 1, 2, 3, 4, 5, 6 years or longer).

As used herein, the term "administration" refers to a method of providing a dosage of compound or composition. The compound and/or composition used in the method described herein may be administered through intravenous (e.g. by intravenous infusion), subcutaneous, intramuscular, intradermal, transdermal, intraarterial, intraabdominal, intralesional, intracranial, intraarticular, intraprostatic, intrapleural, intratracheal, intranasal, intravitreal, intravaginal, intrarectal, intratumoral, intraperitoneal, subconjunctival, intravesical, mucosal, intrapericardial, intraumbilical, intraocular, oral, or topical routes, by suction, by injection, by infusion, by continuous infusion, by localized perfusion directly bathing the target cells, by catheter, by irrigation, as a cream, or as a lipid composition. The method of administration may vary depending on various factors (e.g. the compound or composition to be administered and the severity of the condition, disease, or disorder to be treated).

An immune checkpoint inhibitor and a pharmaceutical formulation including the immune checkpoint inhibitor may be administered on different schedules to a subject depending on some factors to consider, such as the specific goal or purpose of the method; the age and size of a subject; and the general health status of a subject. Generally, the immune checkpoint inhibitor and the pharmaceutical formulation may be administered once, or two, three, four, five, six or more times over the course of treatment or inhibition. The timing between doses on the dosing schedule may fall in the range of days, weeks, months, or years, and includes administration once every 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or more weeks. The immune checkpoint inhibitor may be administered in the same amount or different amounts at each dosing on the dosing schedule. The identity of the immune checkpoint inhibitor may also be different for each dosing on the dosing schedule, or may remain the same.

In the methods of the present invention, the immune checkpoint inhibitor or the pharmaceutical formulation including the immune checkpoint inhibitor is administered in a "therapeutically effective amount" to a subject. A therapeutically effective amount may vary from subject to subject. However, a therapeutically effective amount is an amount sufficient to achieve the goal or purpose of the method, be that inhibition or treatment. By way of example, the therapeutically effective amount of the immune checkpoint inhibitor used in the methods of the present invention is typically about 0.1 µg to about 10,000 µg per kg body weight of a subject receiving the peptide. The therapeutically effective amount of the immune checkpoint inhibitor may also be about 0.5 µg to about 5,000 µg, about 1 µg to about 500 µg, about 10 µg to about 200 µg, about 1 µg to about 800 µg, about 10 µg to 1,000 µg, about 50 µg to about 5,000 µg, about 50 µg to about 500 µg, about 100 µg to about 1,000 µg, about 250 µg to about 2,500 µg, about 500 µg to about 2,000 µg, about 10 µg to about 800 µg, about 10 µg to about 1,000 µg, about 1 µg to about 300 µg, or about 10 µg to about 300 µg per kg body weight of a subject.

Patients in critical condition, such as terminal cancer patients, may be treated with targeted therapy according to specific genetic tests such as EGFR, ALK, ROS1, etc. When targeted therapy is applied for about 1 year, drug resistance may occur, and also, the tumor becomes small and fibrosis occurs around the cancer, so there are many cases in which it is not possible to collect cancer cells even though a tissue test is performed. Moreover, it is difficult to perform a tissue test for prescribing cancer immunotherapy or to monitor a therapeutic response after prescription because terminal cancer patients cannot undergo surgery and repeated tissue testing is difficult.

In the present invention, a biomarker is developed that may show the same diagnostic effect as a tissue test only with a blood test in patients for whom it is difficult to perform such a tissue test. Thereby, cancer immunotherapy may be selected with high accuracy when expression of the immune checkpoint protein and expression of the lymphoid or myeloid-specific protein are simultaneously confirmed.

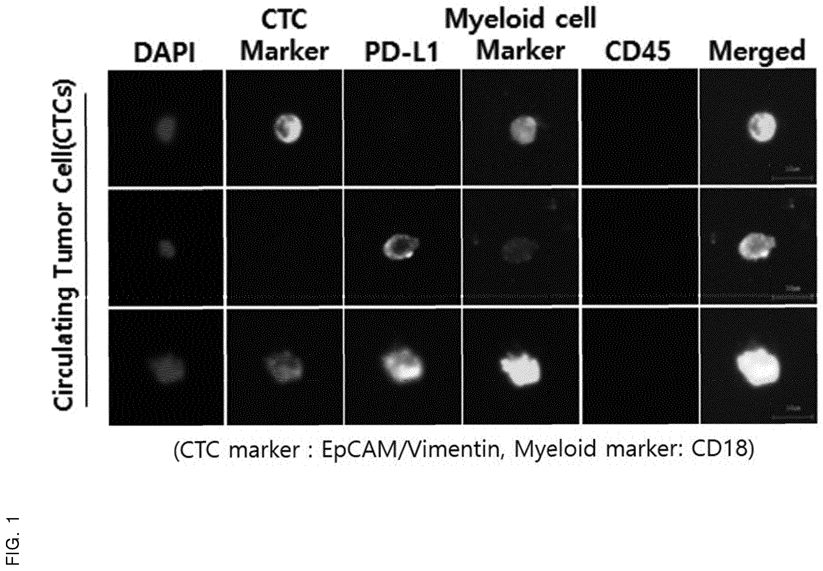

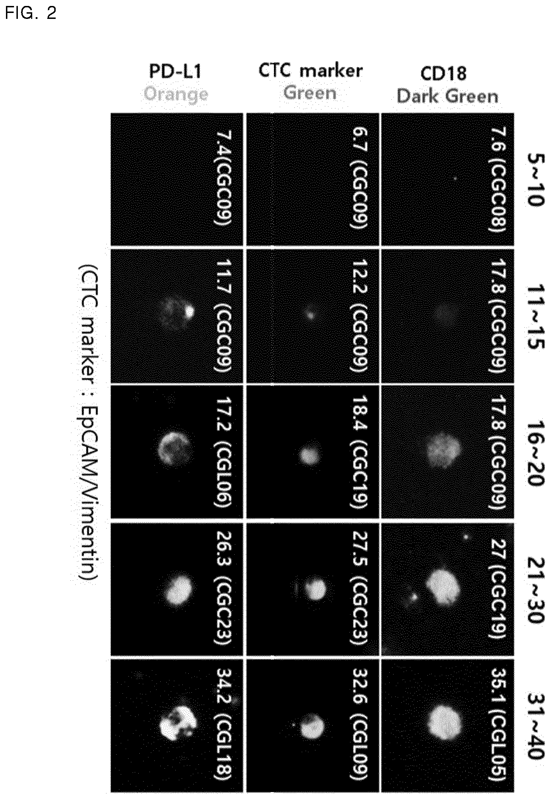

As confirmed in an embodiment of the present invention, when CTCs isolated from the blood are stained with PD-L1 and CD18 antibodies and expression levels thereof are measured, the rate of obtaining the same result as the tissue test result is drastically increased compared to a method of confirming only PD-L1 expression (FIGs. 1 and 2).

Accordingly, an aspect of the present invention pertains to:
a method of selecting a patient for application of cancer immunotherapy drug, including:
(a) confirming expression of an immune checkpoint protein and a lymphoid or myeloid-specific protein in isolated circulating tumor cells; and
(b) classifying a patient in whom immune checkpoint protein expression is confirmed as positive and lymphoid or myeloid-specific protein expression is confirmed as positive as a patient to whom cancer immunotherapy drug is applicable.

In the present invention, the meaning of protein expression being "positive" is that the presence of the protein in circulating tumor cells is confirmed by various methods known to those skilled in the art, preferably by fluorescence intensity, but the present invention is not limited thereto.

In the present invention, determining the protein expression as positive may be based on a specific cut-off value in consideration of the number of cells expressing each or all of proteins and the intensity of expression (fluorescence intensity) for each protein, or based on high or low expression level compared to the reference population, but the present invention is not limited thereto.

In the present invention, the meaning of protein expression being "negative" is that the presence of the protein in circulating tumor cells is not confirmed by various methods known to those skilled in the art, preferably by fluorescence intensity, but the present invention is not limited thereto.

In the present invention, determining the protein expression as negative may be based on a specific cut-off value in consideration of the number of cells not expressing each or all of proteins and the intensity of expression (fluorescence intensity) for each protein, or based on high or low expression level compared to the reference population, but the present invention is not limited thereto.

In the present invention, the immune checkpoint protein may be used without limitation, so long as it is a protein involved in an immune checkpoint-related signaling pathway, and the immune checkpoint protein is preferably selected from the group consisting of CD27, CD28, CD40, CD122, CD137, OX40, GITR, ICOS, A2AR, B7-H3, BY-H4, CTLA-4, IDO, KIR, LAG3, NOX2, PD-1, PD-L1, TIM-3, VISTA, and SIGLEC7, is more preferably selected from the group consisting of PD-1, PD-L1, and CTLA-4, and is most preferably PD-L1, but the present invention is not limited thereto.

In the present invention, the lymphoid or myeloid-specific protein may be used without limitation, so long as it is a protein expressed in lymphoid or myeloid cells, and the lymphoid or myeloid-specific protein is preferably selected from the group consisting of CD18, CD29, CD61, CD104, ITGB5, ITGB6, ITGB7, ITGB8, CD49a, CD49b, CD49c, CD49d, CD49e, CD49f, ITGA7, ITGA8, ITGA9, ITGA10, ITGA11, CD11D, CD103, CD11a, CD11b, CD51, CD41, CD11c, CD64, CD32, CD16a, CD16b, CD23, CD89, FcRn, FcεRI, and Fcα/µR, is more preferably selected from the group consisting of CD18, CD16, CD29, CD61, CD104, CD32, CD11b, and CD64, and is most preferably CD18, but the present invention is not limited thereto.

In the present invention, the circulating tumor cells may be isolated by:
(i) obtaining blood from a cancer patient; and
(ii) isolating cancer cells from the blood using a biochip.

In the present invention, the circulating tumor cells (CTC) are tumor cells found in the peripheral blood of patients with malignant tumors. The circulating tumor cells are very rare and the available amount thereof is very limited. Techniques in the detection and characterization of circulating tumor cells include, but are not limited to, multiplex reverse transcription-quantitative polymerase chain reaction methods, imaging-based approaches, and microfiltration and microchip devices. The circulating tumor cells may act as a tumor biomarker that may inevitably provide individualized treatment and follow-up observation after treatment with a liquid biopsy specimen. Moreover, the circulating tumor cells may be used as, but not limited to, a target for understanding biological characteristics of tumors and dissemination of tumor cells.

The biochip is a hybrid device made in the form of an existing semiconductor chip by integrating and combining materials of biological origin, such as DNA, proteins, enzymes, antibodies, microorganisms, animal and plant cells and organs, neurons, etc. into a solid substrate made of an inorganic material such as a semiconductor, and is a tool or device that uses inherent functions of biomolecules to obtain biological information such as gene expression patterns, gene binding, protein distribution, and the like, or to speed up biochemical processes and reactions or information processing.

A high-density microchip is a biochip capable of separating a material having a specific size based on the working principle of the biochip.

The high-density microchip is based on a difference in size of blood cells and is capable of capturing circulating tumor cells with a recovery rate of about 90% within 10 minutes.

In an embodiment of the present invention, the pore size of the high-density microchip is preferably 5.5 to 8.5 µm, more preferably 6.5 to 7.5 µm. If the pore size thereof is less than 5.5 µm, erythrocytes and leukocytes cannot pass through the chip and are caught on the chip and are not removed. On the other hand, if the pore size thereof is greater than 8.5 µm, the pores are larger than the circulating tumor cells, and thus the circulating tumor cells may pass through the chip, making it impossible to selectively collect the circulating tumor cells. In an embodiment of the present invention, the pores of the high-density microchip may have a circular shape, a rectangular shape, or an elliptical shape, preferably a rectangular shape.

In an embodiment of the present invention, the pores of the high-density microchip may be arranged in a regular pattern. In another embodiment of the present invention, the high-density microchip may be specifically made of stainless steel, nickel, aluminum, or copper. These pores may be formed by etching using MEMS (microelectromechanical system) technology.

The distance between two adjacent pores among the pores may be narrower than the diameter of circulating tumor cells. In an embodiment of the present invention, the distance between two pores may be 45 to 65% of the diameter of circulating tumor cells. The high-density microchip is not deformed by the pressure of blood or a solution flowing through the passage. The blood is discharged to the outside through the pores, and circulating tumor cells in the blood do not pass through the pores but remain on the surface thereof.

Non-target cells, namely erythrocytes having high strain compared to circulating tumor cells, easily pass through the pores.

After filtration of circulating tumor cells, circulating tumor cells may be discharged outside by supplying the solution in a reverse or forward direction. In an embodiment of the present invention, the solution may be supplied in a reverse direction to minimize damage to circulating tumor cells. The solution may be supplied using a syringe, syringe pump, plunger pump, or the like. In an embodiment of the present invention, the solution may be composed of a blood diluent, water, and an acid for dilution. The circulating tumor cells that are discharged outside by supply of the solution may be collected conveniently in a container, for example, a test tube, a culture dish, etc.

Also, circulating tumor cells having a diameter of 7.5 to 15 µm pass through a tube having a diameter of 8 µm when a pressure of about 100 mmHg is applied. Circulating tumor cells having a diameter of 15 µm that pass through a tube having a diameter of 8 µm have a strain of about 53%. When circulating tumor cells having a diameter of 7.5 µm are discharged outside through back washing, particularly by allowing the solution to flow in the opposite direction to the flow of blood, the distance between two pores is preferably set to 4 µm or less in consideration of the strain of circulating tumor cells. For example, non-small cell lung cancer and breast cancer are known to have cancer cells having a diameter of about 40 µm. Taking into consideration the strain of circulating tumor cells having a diameter of 40 µm during back washing, the distance between two pores is preferably set to 21 µm or less. When circulating tumor cells having a diameter of 7.5 µm are present between two pores spaced apart by a distance of greater than 4 µm, circulating tumor cells may not be detached from the surface while being deformed by the flow of the solution. In addition, cancer cells having a diameter of 40 µm may not be detached from the surface between two pores spaced apart by a distance of greater than 21 µm. In consideration of the pressure of the solution in the high-density microchip used in the present invention, the distance between two pores is 45 to 65% of the diameter of circulating tumor cells. The circulating tumor cells having a diameter of 7.5 µm may not be detached from the surface between two pores by back washing when the distance therebetween exceeds 65%, namely about 4.9 µm. The circulating tumor cells having a diameter of 40 µm may not be detached from the surface between two pores by back washing when the distance therebetween exceeds 65%, namely 26 µm. When the pressure of the solution is increased to forcibly detach circulating tumor cells from the surface between two pores, circulating tumor cells are damaged and the collection rate of living circulating tumor cells is lowered. If the distance between pores for circulating tumor cells having a diameter of 7.5 µm is less than 45%, namely about 3.375 µm, the high-density microchip is likely to be damaged by the flow of blood and solution.

In an embodiment of the present invention, the high-density microchip allows the sample to repeatedly pass therethrough. Specifically, circulating tumor cells may be separated once from the high-density microchip, followed by separation once again by loading the separated circulating tumor cells into the high-density microchip, and such a separation process may be repeated.

In an embodiment of the present invention, separation of circulating tumor cells through the high-density microchip may be performed by gravity rather than by applying a certain artificial pressure after loading a solution containing circulating tumor cells into the high-density microchip. Separation of circulating tumor cells through the high-density microchip used in the present invention may minimize damage caused by artificial pressure to circulating tumor cells, thereby allowing the same to maintain the existing state in the patient's body.

In an embodiment of the present invention, the high-density microchip may be coated with a specific material in order to minimize damage to circulating tumor cells by the high-density microchip when circulating tumor cells are separated or to make repeated use of the high-density microchip more efficient, or to more efficiently collect circulating tumor cells. The specific material may be an antibody capable of specifically binding to circulating tumor cells, and may be any biomaterial that does not physically or chemically damage cells. In an embodiment of the present invention, the specific material may be BSA (bovine serum albumin) or an antibody. The antibody may include, for example, anti-epithelial cell adhesion molecule antibody (anti-EpCAM antibody), anti-cytokeratin antibody (anti-CK antibody), and the like. According to a preferred embodiment of the present invention, the specific material is BSA (bovine serum albumin) .

The BSA solution indicates bovine serum albumin, which is a protein with a molecular weight of about 66.4 kDa and is mainly present in animals. BSA may be added as a cell nutrient for cell culture in biochemistry/biology, and is also often used as a standard material for obtaining a calibration curve in protein quantification. When using a restriction enzyme, a small amount of enzyme (protein) has to be used, and thus BSA may be added to compensate for the protein concentration in the solution. Also, in various biochemical experiments (Western blot, immunocytochemistry, ELISA, etc.), before attaching a specific antibody to a protein to be detected, BSA may be used to prevent nonspecific binding, that is, nonspecific binding of an antibody to an unwanted protein or an unwanted location.

According to an embodiment of the present invention, other biopolymers except circulating tumor cells may be removed by allowing peripheral blood to react with a high-density microchip coated with the BSA solution using a centrifugation process. In an embodiment of the present invention, separation using the high-density microchip may be performed by gravity, particularly under atmospheric pressure of 1000 to 1020 hPa, preferably under atmospheric pressure of 1000 to 1015 hPa, more preferably under atmospheric pressure of 1000 to 1013 hPa.

According to an embodiment of the present invention, the BSA solution may be applied onto the upper and lower surfaces of the high-density microchip or the inner surface of the pores. Preferably, both the upper and lower surfaces of the high-density microchip and the inner surface of the pores are coated therewith.

According to an embodiment of the present invention, the BSA solution coating may have a concentration of 0.05 to 0.15%. According to a preferred embodiment of the present invention, the BSA solution coating has a concentration of 0.08 to 0.012%.

According to an embodiment of the present invention, coating with the BSA solution may be performed for 5 to 15 minutes. According to a preferred embodiment of the present invention, coating with the BSA solution is performed for 8 to 12 minutes.

In the present invention, confirming the expression of the immune checkpoint protein and the lymphoid or myeloid-specific protein may be performed by:
(1) carrying out reaction with a fluorescent marker that specifically binds to circulating tumor cells, a fluorescent marker that specifically binds to an immune checkpoint protein, and a fluorescent marker that specifically binds to a lymphoid or myeloid-specific protein;
(2) receiving optical images in multiple wavelength ranges for the circulating tumor cells, the immune checkpoint protein, and the lymphoid or myeloid-specific protein, reacted with the fluorescent markers;
(3) performing primary filtering by measuring fluorescence intensities of the circulating tumor cells, the immune checkpoint protein, and the lymphoid or myeloid-specific protein in optical images of all or part of the multiple wavelength ranges;
(4) performing secondary filtering by measuring the morphology of the circulating tumor cells in the optical images of all or part of the multiple wavelength ranges; and
(5) performing tertiary filtering by measuring the morphology of the circulating tumor cells in an integrated image obtained by merging all or part of the optical images in the multiple wavelength ranges.

In the present invention, the fluorescent marker that specifically binds to circulating tumor cells is a fluorescent material that may specifically bind to circulating tumor cells themselves or materials present inside or outside cells. According to an embodiment of the present invention, the fluorescent marker capable of identifying circulating tumor cells may specifically bind to cell nuclei or specifically bind to proteins, DNA, RNA, etc. present inside or outside cells. Particularly, DAPI may be used as a fluorescent marker for cell nuclei, and a fluorescent marker that specifically binds to vimentin, which is an intermediate filament protein, may be used, a fluorescent marker that specifically binds to EpCAM (epithelial cell adhesion molecule) and CK (cytokeratin) may be used, and a fluorescent marker that specifically binds to CD45 serving as a non-target cell remover may be used. The fluorescent marker that specifically binds to circulating tumor cells may be composed of a nucleotide, oligonucleotide, peptide, polypeptide, nucleic acid, or protein, and may be an antibody made of protein. The fluorescent marker that specifically binds to circulating tumor cells may be any material that specifically binds to and identifies circulating tumor cells.

The optical image may be obtained using an imaging system. According to an embodiment of the present invention, the imaging system may be a cell imaging system, and the cell imaging system is a system configured such that stained cells are placed on a platform such as a slide glass and observed and imaged at various wavelengths. The cell imaging system may include an automatic cell counting module, a fluorescence intensity analysis module, and a cytology-based cell classification/cognition module. Also, for user convenience functions, a measurement function, a report automatic generation function, and a DB management function may be included as user interfaces. This cell imaging system includes a digital image analyzer configured to discriminate cells, culture fluid, debris, and the like and to discriminate and count cells desired by the user. The cell imaging system used according to an embodiment of the present invention is capable of accurately identifying and counting fluorescently labeled or marker-bound target cells from optical images.

The optical image may be an optical image obtained through reflective light reflected from an object. The cell optical image may be an image of cells and debris on a background output by a cell imaging device. This optical image may be provided as one image file by stitching a plurality of divided images in specific wavelength ranges. Here, optical images in multiple wavelength ranges may include an image in a blue wavelength range, an image in a green wavelength range, and an image in a red wavelength range. The optical image in the blue wavelength range is particularly useful for identifying cell nuclei of circulating tumor cells, and the optical images in the green and red wavelength ranges are particularly useful for identifying cell membranes of circulating tumor cells. In addition, it is possible to identify specific fluorescent markers in various color wavelength ranges.

In the primary filtering or the secondary filtering, if identification of the circulating tumor cells, the immune checkpoint protein, and the lymphoid or myeloid cell-specific protein is not achieved to a desired level, primary filtering or secondary filtering may be performed once again after data filtering of optical image data, and data filtering may be conducted multiple times. Moreover, after primary filtering or secondary filtering, image data may be stored and output.

In addition, the primary filtering and the secondary filtering are performed on the optical image in the first wavelength range, after which at least one of the primary filtering or the secondary filtering may be further performed on the optical image in the second wavelength range different from the first wavelength range.

For example, the first wavelength range may be a blue wavelength range, and the second wavelength range may be a green or red wavelength range. As such, the cell nuclei of circulating tumor cells may be discriminated by performing the primary filtering process and the secondary filtering process on the image in the blue wavelength range. In addition, the cell membranes of circulating tumor cells may be discriminated by performing the primary filtering process on at least one of the image in the green wavelength range or the image in the red wavelength range. Through the primary and secondary filtering processes, it is possible to accurately identify cells from optical images. For example, identification of target cells such as cancer cells and leukocytes may be increased through images in the green and red wavelength ranges.

Also, in the secondary filtering process, the morphology of the circulating tumor cells is measured. Here, the morphology of the circulating tumor cells may include at least one selected from among cell area, cell size (diameter), and circularity.

In an embodiment of the present invention, the measurement of fluorescence intensity of the immune checkpoint protein and the lymphoid or myeloid cell-specific protein is performed by measuring fluorescence intensity in optical images of all or part of multiple wavelength ranges emanating from fluorescent markers that specifically bind to the immune checkpoint protein and the lymphoid or myeloid cell-specific protein, and primary filtering may be performed therefor.

Looking more specifically at the process of performing primary filtering by measuring the fluorescence intensity of circulating tumor cells in addition to the immune checkpoint protein and the lymphoid or myeloid cell-specific protein, primary filtering may be performed by measuring the size of cells in optical images of all or part of multiple wavelength ranges, setting a polygonal or circular region larger by a predetermined ratio or amount than the measured cell size, and measuring the fluorescence intensity of the cells within this region.

The tertiary filtering is performed by measuring the morphology of circulating tumor cells in an integrated image obtained by merging all or part of optical images in multiple wavelength ranges. Here, the morphology of the circulating tumor cells may include at least one selected from among cell area, cell size, and circularity.

The tertiary filtering may be performed on all circulating tumor cells, or may be supplementally conducted on circulating tumor cells that are difficult to identify during the primary and secondary filtering processes. When requiring additional identification, separate data filtering may be carried out, or tertiary filtering may be performed multiple times.

The image analysis may be executed by a computer program. Such a computer program may be implemented using at least one general-purpose or special-purpose computer, such as a processor, a controller, an ALU (arithmetic logic unit), a digital signal processor, a microcomputer, an FPGA (field programmable gate array), a PLU (programmable logic unit), a microprocessor, or any other device capable of executing and responding to instructions.

In the present invention, the fluorescent marker that specifically binds to the immune checkpoint protein may be selected from the group consisting of a PD-1-specific antibody, a PD-L1-specific antibody, and a CTLA-4-specific antibody, but is not limited thereto.

In the present invention, the fluorescent marker that specifically binds to the lymphoid or myeloid protein may be selected from the group consisting of a CD18-specific antibody, a CD16-specific antibody, a CD29-specific antibody, a CD61-specific antibody, a CD104-specific antibody, a CD32-specific antibody, a CD11b-specific antibody, and a CD64-specific antibody, but is not limited thereto.

In the present invention, the immune checkpoint inhibitor may be used without limitation, so long as it is a material that is able to inhibit the function of the immune checkpoint protein, and may be a protein, compound, natural material, DNA, RNA, peptide, etc., and is preferably an antibody, more preferably a monoclonal antibody, much more preferably a human antibody, a humanized antibody, or a chimeric antibody.

In the present invention, the immune checkpoint inhibitor may be at least one selected from the group consisting of a PD-L1 antagonist, a PD-1 antagonist, and a CTLA-4 antagonist, but is not limited thereto.

As used herein, the term "PD-1 antagonist" refers to a molecule that reduces, blocks, inhibits, abrogates, or interferes with signal transduction due to the interaction of PD-1 with at least one binding partner thereof, such as PD-L1 and/or PD-L2. In some embodiments, the PD-1 antagonist is a molecule that inhibits the binding of PD-1 to a binding partner thereof. In a specific embodiment, the PD-1 antagonist inhibits the binding of PD-1 to PD-L1 and/or PD-L2. For example, the PD-1 antagonist includes another molecule that reduces, blocks, inhibits, abrogates, or interferes with signal transduction due to interactions of an anti-PD-1 antibody, an antigen-binding fragment thereof, an immunoadhesin, a fusion protein, an oligopeptide, a small molecule antagonist, a polynucleotide antagonist, and PD-1 with PD-L1 and/or PD-L2. In an embodiment, the PD-1 antagonist reduces a negative signal mediated through a cell surface protein or by a cell surface protein expressed on T lymphocytes and other cells that mediate signaling through PD-1 or PD-L1, such that dysfunctional T-cells are less dysfunctional. In some embodiments, the PD-1 antagonist is an anti-PD-1 antibody.

As used herein, the term "PD-L1 antagonist" refers to a molecule that reduces, blocks, inhibits, abrogates, or interferes with signal transduction due to the interaction of PD-L1 with at least one binding partner thereof, such as PD-1 or B7-1. In some embodiments, the PD-L1 antagonist is a molecule that inhibits the binding of PD-L1 to a binding partner thereof. In a specific embodiment, the PD-L1 antagonist inhibits the binding of PD-L1 to PD-1 and/or B7-1. In some embodiments, the PD-L1 antagonist includes another molecule that reduces, blocks, inhibits, abrogates, or interferes with signal transduction due to interactions of an anti-PD-L1 antibody, an antigen-binding fragment thereof, an immunoadhesin, a fusion protein, an oligopeptide, and PD-L1 with a binding partner thereof, such as PD-1 or B7-1. In an embodiment, the PD-L1 antagonist reduces a negative co-stimulatory signal mediated through a cell surface protein or by a cell surface protein expressed on T lymphocytes that mediate signaling through PD-L1, such that dysfunctional T-cells are less dysfunctional (e.g. enhancement of effector responses to antigen recognition). In some embodiments, the PD-L1 antagonist is an anti-PD-L1 antibody.

As used herein, the term "CTLA4 antagonist" refers to a molecule that reduces, blocks, inhibits, abrogates, or interferes with signal transduction due to the interaction of CTLA4 with at least one binding partner thereof, such as B7-1. In some embodiments, the CTLA4 antagonist is a molecule that inhibits the binding of CTLA4 to a binding partner thereof. In a specific embodiment, the CTLA4 antagonist inhibits the binding of CTLA4 to B7-1. In some embodiments, the CTLA4 antagonist includes another molecule that reduces, blocks, inhibits, abrogates, or interferes with signal transduction due to interactions of an anti-CTLA4 antibody, an antigen-binding fragment thereof, an immunoadhesin, a fusion protein, an oligopeptide, and CTLA4 with a binding partner thereof, such as B7-1. In an embodiment, the CTLA4 antagonist reduces a negative co-stimulatory signal mediated through a cell surface protein or by a cell surface protein expressed on T lymphocytes that mediate signaling through CTLA4, such that dysfunctional T-cells are less dysfunctional (e.g. enhancement of effector responses to antigen recognition). In some embodiments, the CTLA4 antagonist is an anti-CTLA4 antibody.

In the present invention, the immune checkpoint inhibitor may be at least one selected from the group consisting of pembrolizumab (Keytruda), nivolumab (Opdivo), cemiplimab (Lybtayo), atezolizumab (Tecentriq), avelumab (Bacencio), durvalumab (Imfinzi), ipilimumab (Yervoy), and tremelimumab, but is not limited thereto.

In the present invention, the cancer may be selected from the group consisting of lung cancer, kidney cancer, bladder cancer, breast cancer, colorectal cancer, ovarian cancer, pancreatic cancer, gastric carcinoma, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycosis fungoides, Merkel cell cancer, and hematological malignancies, and is preferably lung cancer, most preferably non-small cell lung cancer, but is not limited thereto.

Another aspect of the present disclosure (not part of the present invention) pertains to:
a method of treating cancer, including:
(a) confirming expression of an immune checkpoint protein and a lymphoid or myeloid-specific protein in isolated circulating tumor cells; and
(b) administering an immune checkpoint inhibitor when immune checkpoint protein expression is positive and lymphoid or myeloid-specific protein expression is positive.

Disclosed herein, but not as claimed invention is:
a method of determining susceptibility to an immune checkpoint inhibitor, including:
(a) confirming expression of an immune checkpoint protein and a lymphoid or myeloid-specific protein in isolated circulating tumor cells; and
(b) determining that there is susceptibility to an immune checkpoint inhibitor when immune checkpoint protein expression is positive and lymphoid or myeloid-specific protein expression is positive.

Disclosed herein, but not as claimed invention is:
a method of selecting cancer immunotherapy for a cancer patient, including:
(a) confirming expression of an immune checkpoint protein and a lymphoid or myeloid-specific protein in isolated circulating tumor cells; and
(b) selecting an immune checkpoint inhibitor as therapy when immune checkpoint protein expression is positive and lymphoid or myeloid-specific protein expression is positive.

Disclosed herein, but not as claimed invention is:
a pharmaceutical composition for cancer treatment, including an immune checkpoint inhibitor for treating a subject with positive immune checkpoint protein expression and positive lymphoid or myeloid-specific protein expression.

Disclosed herein, but not as claimed invention is:
the use of an immune checkpoint inhibitor for the manufacture of a medicament for treating a subject with positive immune checkpoint protein expression and positive lymphoid or myeloid-specific protein expression.

### Examples

A better understanding of the present invention may be obtained through the following examples. These examples are merely set forth to illustrate the present invention, and are not to be construed as limiting the scope of the present invention, as will be obvious to those skilled in the art.

### Example 1. Isolation of CTCs from blood

This study was approved by the Institutional Review Board (IRB), and tissue and blood samples from 20 lung cancer patients (Table 1) and 20 inflammatory patients (Table 2) were used after obtaining informed consent.

**[Table 1]**

| Information on lung cancer patients | | | | | | |
|---|---|---|---|---|---|---|
| **No** | **Subject No** | **Age** | **Sex** | **Stage** | **Metastasis** | **Cancer type** |
| **1** | CGL05 | 59 | M | T4N0M1b | Y | Adenocarcinoma |
| **2** | CGL06 | 59 | F | T2aN0/3M1c | Y | Adenocarcinoma |
| **3** | CGL07 | 68 | M | T4N3M1c | Y | Adenocarcinoma |
| **4** | CGL08 | 65 | M | T2aN3M1c | Y | Adenocarcinoma |
| **5** | CGL09 | 52 | M | T2aN2M1a | Y | Adenocarcinoma |
| **6** | CGL14 | 71 | F | T2aN3M0 | N | NSCLC NOS |
| **7** | CGL15 | 42 | M | T1bN3M1c | Y | Adenocarcinoma |
| **8** | CGL16 | 74 | M | T3N2M1c | Y | SqCC |
| **9** | CGL18 | 62 | M | T3N2M0 | N | SqCC |
| **10** | CGL19 | 68 | M | T4N3M1c | Y | Adenocarcinoma |
| **11** | CGL21 | 59 | M | T3N3M1c | Y | Adenocarcinoma |
| **12** | CGL22 | 55 | M | T1cN2Mo | Y | Small cell carcinoma |
| **13** | CGL23 | 62 | F | T1cN1/3M1a | N | Adenocarcinoma |
| **14** | CGL24 | 54 | F | T4N3M1b | Y | Adenocarcinoma |
| **15** | CGL25 | 47 | M | T2N3M1c | Y | Adenocarcinoma |
| **16** | CGL26 | 74 | F | T4N3M1b | Y | Adenocarcinoma |
| **17** | CGL27 | 75 | M | T4N2M0 | N | SqCC |
| **18** | CGL28 | 67 | M | T1cN3M1c | Y | Adenocarcinoma |
| **19** | CGL29 | 70 | M | T3N2M1c | Y | Adenocarcinoma |
| **20** | CGL30 | 66 | M | T4N1M1a | Y | Adenocarcinoma |

| | | | | | | |
|---|---|---|---|---|---|---|
| (NOS: not other specified, SqCC: squamous cell carcinoma) | | | | | | |

**[Table 2]**

| Information on inflammatory patients | | | | |
|---|---|---|---|---|
| **No** | **Subject No** | **Age** | **Sex** | **Disease** |
| **1** | CGC07 | 61 | F | Bacterial |
| **2** | CGC08 | 53 | F | NTM disease |
| **3** | CGC09 | 66 | M | Tuberculosis |
| **4** | CGC10 | 60 | M | Bacterial |
| **5** | CGC11 | 54 | M | Parasitic |
| **6** | CGC18 | 66 | F | NOS |
| **7** | CGC19 | 78 | F | NOS |
| **8** | CGC20 | 71 | M | NTM disease |
| **9** | CGC21 | 59 | F | NOS |
| **10** | CGC22 | 69 | M | NOS |
| **11** | CGC23 | 66 | M | NOS |
| **12** | CGC24 | 54 | M | Tuberculosis |
| **13** | CGC25 | 68 | F | Bacterial |
| **14** | CGC26 | 59 | F | NTM disease |
| **15** | CGC27 | 46 | M | NOS |
| **16** | CGC28 | 59 | F | NTM disease |
| **17** | CGC29 | 38 | M | Bacterial |
| **18** | CGC30 | 56 | F | NTM disease |
| **19** | CGC32 | 60 | M | Tuberculosis |
| **20** | CGC33 | 65 | M | NTM disease |

| | | | | |
|---|---|---|---|---|
| (NOS: not other specified, NTM: nontuberculous mycobacterial) | | | | |

CTCs were isolated from the patient's blood using a Smart Biopsy^{™} cell Isolation kit (CytoGen) and a Smart Biopsy^{™} Cell Isolator (CytoGen) according to the manufacturer's instructions. A high-density microchip for use in the Smart Biopsy^{™} Cell Isolator was a 5 µm pore size chip to allow target cells larger than 5 µm to be collected on the chip. The microchip is designed to selectively collect cells having a specific size because the pore size thereof is controllable (US10,502,668, KR10-1254675).

### Smart Biopsy^{™} Cell Isolator (CIS030)

- A Smart Biopsy^{™} Cell Isolator is a device configured to automatically perform mixing, transport, and filtering with an ADP (air displacement pipette) by recognizing the height of the target (peripheral blood mononuclear cell, PBMC) from the solution. The system of this device is composed of an X-Y main axis, an ADP, a chip handler, and a consumable table. Basically, a Smart Biopsy^{™} Cell Isolator operates according to the manual using software.

### Example 2. Marker screening for selection of isolated CTCs

### 2-1. CTC staining method

### Smart Biopsy^{™} IF Stainer (CST030)

- Using an automatic immunostaining device, simultaneous staining of a total of 12 slides is possible. The system of this device is composed of an X-Y main axis module, a 1 ml pipette (ADP) module, a covering module, a consumable part module, and a vision part module, and also includes a consumable table. A Smart Biopsy^{™} Cell Isolator operates according to the manual using self-made software.

### CTC staining method

### [Smart Biopsy^{™} IF Stainer method]

1. Place slides with fixed cells and necessary antibodies (e.g. EpCAM, Vimentin, PD-L1, CD18, CD45 antibodies) in respective buffer tubes in a Smart Biopsy^{™} IF Stainer, followed by operation according to the manual.
2. Automatically perform washing, blocking, primary antibody/secondary antibody staining, and mounting for a total of 6 hours (based on 12 slides).
3. Perform all staining processes using a Smart Biopsy^{™} IF Stainer according to the [Manual method] below.

### [Manual method]

1. Treat a sample (slide) with 50 µl of 0.2% Triton X-100 for 10 minutes, followed by washing three times for 5 minutes using PBS.
2. Perform blocking for 1 hour using 1% BSA to reduce nonspecific binding.
3. Dilute each of necessary antibodies (e.g. Anti-CD45 for negative selection of immune cells, Anti-CD18 for negative selection of lymphoid cells, and PD-L1 antibody for positive selection) in 1% BSA, followed by treatment for 1 hour (Primary antibody treatment).
4. Dilute secondary antibodies (goat Anti-mouse Alexa Fluor 647 and goat Anti-rabbit Alexa Fluor 546) having two different wavelengths in 1% BSA, followed by storage at room temperature for 1 hour (Secondary antibody treatment).
5. Perform blocking for 1 hour to inhibit cross-reaction with a lung cancer CTC-specific antibody (PD-L1).
6. Store an anti-EpCAM Alexa Fluor 488 conjugated antibody, which is a CTC marker, at room temperature for 1 hour.
7. Perform washing three times for 5 minutes using PBS.
8. Perform a DAPI staining process, followed by a mounting process using a cover glass.

### 2-2. Marker screening for CTC selection

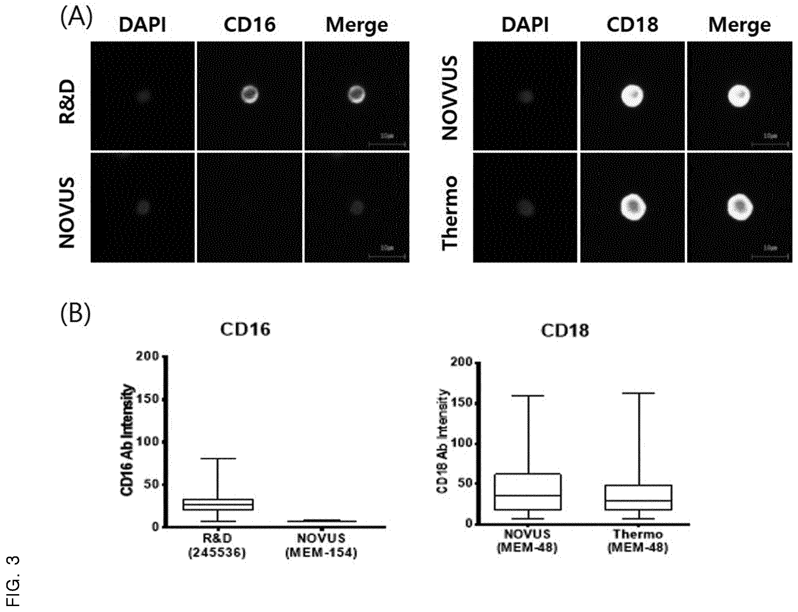

In order to more clearly identify CTCs in the blood, the expression levels of markers were confirmed using anti-Rabbit IgG, Alexa Fluor 546 (A-11010/2 mg/ml) and anti-mouse IgG, Alexa Fluor 546 (A-11003/2 mg/ml) from Thermo Fisher as secondary antibodies as well as antibodies in Table 3 below in PBMCs in the patient's blood, indicating that the expression level of CD18 was the highest among markers, as shown in FIG. 3.

**[Table 3]**

| **Product name** | **Manufacturer** | **Cat No.** | **Stock con.** | **Working rate** |
|---|---|---|---|---|
| CD18 (M) | NOVUS | MAB1730 | 1 mg/ml | 1:10 ~ 1:500 |
| CD18 (M) | Thermo | 37-1300 | 0.5 mg/ml | 1:10 ~ 1:500 |
| CD18 (R) | CST | 73663 | 60 µg/ml | 1:10 ~ 1:500 |
| CD16 (M) | R&D | MAB2546-500 | 0.5 mg/ml | 1:10 ~ 1:500 |
| CD16 (M) | NOVUS | NB500-378 | 1 mg/ml | 1:10 ~ 1:500 |

### Example 3. Selected marker-based CTC staining

The cells (approximately 500 to 10,000 or more) isolated by the Isolator were subjected to a cytospin process, which is a cell centrifugation process, and fixed onto slides for staining.

The characteristics of isolated CTCs were confirmed using CD45 for negative selection of immune cells and EpCAM/Vimentin, PD-L1, and CD18 markers for positive selection of CTCs.

As antibodies, EpCAM/Vimentin, PD-L1, CD18 antibody, and CD45 antibody were used, and antibody information is shown in Table 4 below. Then, DAPI (4',6-diamidino-2-phenylindole) capable of staining cell nuclei was added and cell staining was performed using a Smart Biopsy^{™} IF Stainer (CST030, CytoGen) according to the manufacturer's instructions.

**[Table 4]**

| Antibody information | | | | |
|---|---|---|---|---|
| **Product name** | **Manufacturer** | **Cat No.** | **Stock con.** | **Working rate** |
| PD-L1 22C3 | Dako | M3653 | 150 mg/ml | 1:10 ~ 1:500 |
| CD18 (M) | Thermo | 37-1300 | 0.5 mg/ml | 1:10 ~ 1:500 |
| CD18 (R) | CST | 73663 | 60 µg/ml | 1:10 ~ 1:500 |
| EpCAM-488 | CST | 5198S | 100 µg/ml | 1:10 ~ 1:500 |
| Vimentin-488 | CST | 9854S | 200 µg/ml | 1:10 ~ 1:500 |
| CD45 | BD | 561864 | - | 50 µl |

### Example 4. Analysis of CTC fluorescence image

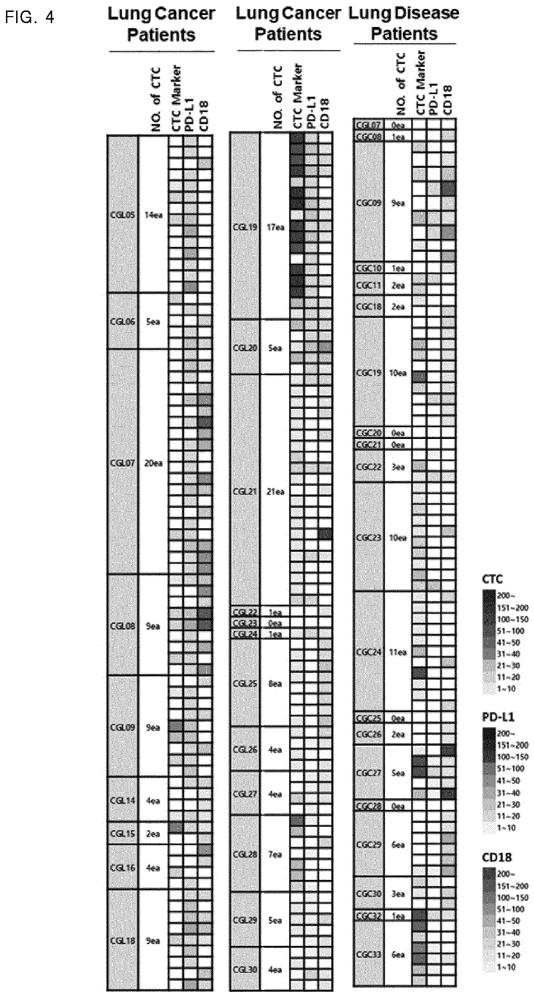

Using a Smart Biopsy^{™} Cell Image Analyzer (CytoGen), images of slides loaded with fluorescently labeled CTCs were obtained, and the number and fluorescence intensity of cells expressing EpCAM/Vimentin, PD-L1, and CD18 were measured (FIGs. 1, 2, and 4).

CTCs expressing PD-L1 and CD18 were identified using blood samples from inflammatory patients and lung cancer patients (FIG. 1). In the present invention, a CD18 marker was additionally introduced in order to increase the accuracy upon determining cancer immunotherapy through PD-L1 expression analysis.

Therefore, the ratios of EpCAM&Vim(+)/PD-L1(+) and EpCAM&Vim(+)/PD-L1(+)/CD18(+) cells were compared based on total CTCs, namely EpCAM&Vim(+)-expressing cells. Also, it was possible to increase the accuracy by analyzing the PD-L1 fluorescence intensity and the number of pure PD-L1(+) cells.

### Example 5. Analysis of CTC-based PD-L1 expression

### 5-1. Analysis of lung cancer patient group

CTC-based PD-L1 expression was analyzed in the lung cancer patient group, and the results thereof were compared with tissue.

Among CTCs expressing EpCAM&Vim(+), the ratios of EpCAM&Vim(+)/PD-L1(+) and EpCAM&Vim(+)/PD-L1(+)/CD18(+) cells were determined and were compared with tissue TPS values.

Analysis of PD-L1 expression in tissue from 20 lung cancer patients was performed with DAKO pharmdx clone 22c3 and VENTANA SP263, and PD-L1(+) and PD-L1(+)/CD18(+) expression was also compared in CTCs from the same patients (Table 5). The number of PD-L1-positive cells of circulating tumor cells based on liquid biopsy of lung cancer patients was counted, and target antibody-specific antibody sensitivity and specificity were calculated and then compared with TPS in the clinical field for analysis, so that applicability of the relevant effective indicators was compared, and the performance thereof was verified.

**[Table 5]**

| Marker-expressing cell ratio in lung cancer patient group (%) | | | | | | | |
|---|---|---|---|---|---|---|---|
| **No** | **Subject No** | **22C3** | **TPS (%)** | **SP263** | **TPS(%)** | **EpCAM&Vim(+)/ PD-L1(+) (%)** | **EpCAM&Vim(+)/ PD-L1(+)/CD18(+) (%)** |
| **1** | CGL05 | N | <1 | P | 30 | 100 | 0 |
| **2** | CGL06 | N | 0 | N | 0 | 0 | 0 |
| **3** | CGL07 | P | 1 | N | <1 | 100 | 0 |
| **4** | CGL08 | P | 100 | P | 100 | 100 | 75 |
| **5** | CGL09 | N | <1 | N | 0 | 100 | 0 |
| **6** | CGL14 | P | 90 | P | 100 | 100 | 100 |
| **7** | CGL15 | N | 0 | N | 0 | 0 | 0 |
| **8** | CGL16 | P | 90 | P | 90 | 0 | 0 |
| **9** | CGL18 | P | 95 | P | 95 | 100 | 100 |
| **10** | CGL19 | P | 1 | P | 1 | 100 | 62.5 |
| **11** | CGL21 | N | <1 | N | 1 | 20 | 15 |
| **12** | CGL22 | N | 0 | N | 0 | 0 | 0 |
| **13** | CGL23 | N | 0 | N | 0 | 0 | 0 |
| **14** | CGL24 | P | 70 | P | 80 | 100 | 100 |
| **15** | CGL25 | N | 0 | N | 0 | 0 | 0 |
| **16** | CGL26 | N | 0 | P | 5 | 25 | 0 |
| **17** | CGL27 | P | 5 | P | 5 | 100 | 100 |
| **18** | CGL28 | N | 0 | N | 0 | 40 | 0 |
| **19** | CGL29 | P | 90 | P | 90 | 100 | 33.3 |
| **20** | CGL30 | N | 0 | N | 0 | 100 | 50 |

Thereby, when CD18 was additionally used compared to when the PD-L1 marker was used alone, the concordance with tissue was confirmed to remarkably increase. Specifically, when comparing PD-L1(+) cells (%) based on TPS (%) of 22C3, the approximate concordance increased from 65% to 80%. Therefore, in PD-L1 expression analysis for determining CTC-based cancer immunotherapy, additional introduction of a CD18 marker increased the accuracy and the concordance with tissue.

However, upon discordance with tissue, the number and fluorescence intensity of PD-L1(+) CTCs were additionally analyzed, compared, and verified (Table 7).

### 5-2. Analysis of inflammatory patient group

PD-L1 expression was verified in the inflammatory patient group in the same manner as in the lung cancer patient group.

**[Table 6]**

| Marker-expressing cell ratio in inflammatory patient group (%) | | | |
|---|---|---|---|
| **No** | **Subject No** | **EpCAM&Vim(+)/PD-L1(+) (%)** | **EpCAM&Vim(+)/PD-L1(+)/CD18(+) (%)** |
| **1** | CGC07 | 0 | 0 |
| **2** | CGC08 | 0 | 0 |
| **3** | CGC09 | 25 | 25 |
| **4** | CGC10 | 0 | 0 |
| **5** | CGC11 | 50 | 0 |
| **6** | CGC18 | 0 | 0 |
| **7** | CGC19 | 0 | 0 |
| **8** | CGC20 | 0 | 0 |
| **9** | CGC21 | 0 | 0 |
| **10** | CGC22 | 50 | 50 |
| **11** | CGC23 | 10 | 0 |
| **12** | CGC24 | 0 | 0 |
| **13** | CGC25 | 0 | 0 |
| **14** | CGC26 | 0 | 0 |
| **15** | CGC27 | 75 | 75 |
| **16** | CGC28 | 0 | 0 |
| **17** | CGC29 | 100 | 100 |
| **18** | CGC30 | 0 | 0 |
| **19** | CGC32 | 100 | 100 |
| **20** | CGC33 | 50 | 16.7 |

Thereby, when the PD-L1 marker was used alone, the expression level of PD-L1 was also high in inflammatory cells from inflammatory patients. However, when CD18(+) was used as an additional marker, the ratio of cells expressing PD-L1 in inflammatory cells decreased (Table 6). When the case in which the ratio of PD-L1(+)-expressing cells was 50% or more is considered positive, the ratio of PD-L1(+)-expressing cells was confirmed to decrease from about 30% to about 20%.

In Table 6, the ratio of PD-L1(+) cells was determined by measuring the ratios of EpCAM&Vim(+)/PD-L1(+) and EpCAM&Vim(+)/PD-L1(+)/CD18(+) cells based on total CTCs, namely EpCAM&Vim(+)-expressing cells.

### Example 6. Analysis of number and fluorescence intensity of PD-L1(+) cells

Upon discordance with tissue in Table 5 and upon detection of PD-L1(+)-expressing cells in inflammatory patients in Table 6, the number and fluorescence intensity of PD-L1(+) CTCs were additionally analyzed and a method of determining CTC-based cancer immunotherapy was established and verified.

For inflammatory patients, the number and fluorescence intensity of PD-L1(+)/CD18(+) cells were confirmed in 8 patients in which PD-L1(+) cell expression was detected (Table 7).

**[Table 7]**

| Number and fluorescence intensity of PD-Ll-expressing cells in inflammatory patient group | | | | | | | | | |
|---|---|---|---|---|---|---|---|---|---|
| **Subject No** | | CGC09 | CGC11 | CGC22 | CGC23 | CGC27 | CGC29 | CGC32 | CGC33 |
| **PD-L1 (+)** | Cell ratio (%) | 25 | 50 | 50 | 10 | 75 | 100 | 100 | 50 |
| **PD-L1(+)/CD18(+)** | Cell ratio (%) | 25 | 0 | 50 | 0 | 75 | 100 | 100 | 16.7 |
| | Cell number | 1 | 0 | 1 | 0 | 3 | 1 | 1 | 1 |
| | Average fluorescence intensity | 11.7 | - | 14.6 | - | 10.57 | 8.9 | 11 | 8.9 |

Thereby, the number of cells was 1 and the average fluorescence intensity was 11.2 in most cases.

For the lung cancer patient group, also, the number and fluorescence intensity of PD-L1(+)/CD18(+) cells were confirmed in patients with concordant tissue TPS and CTC results and in patients with discordant results (Table 8).

**[Table 8]**

| Number and fluorescence intensity of PD-Ll-expressing cells in lung cancer patient group | | | | | | | | | |
|---|---|---|---|---|---|---|---|---|---|
| **Subject No** | | CGL05 | CGL08 | CGL09 | CGL19 | CGL21 | CGL27 | CGL29 | CGL30 |
| **TPS / SP263) (%)** | | <1 /30 | 100/100 | <1 / 0 | 1 / 1 | <1 / 1 | 5 / 5 | 90 / 90 | 0 / 0 |
| **PD-L1(+)** | Cell ratio (%) | 100 | 100 | 100 | 100 | 20 | 100 | 100 | 100 |
| **PD-L1(+) /CD18 (+)** | Cell ratio (%) | 0 | 75 | 0 | 62.5 | 15 | 100 | 33.3 | 50 |
| | Cell number | 0 | 3 | 0 | 10 | 3 | 1 | 1 | 1 |
| | Average fluorescence intensity | | 24.83 | | 19.41 | 14.8 | 8.9 | 9. 2 | 10.3 |

Thereby, when both tissue and CTC results were positive, as in CGL08 and CGL19, the number of cells was 3 to 10 and the fluorescence intensity was 19.41 to 24.88. In addition, when both tissue and CTC results were negative, as in CGL21, the number of cells was 3 and the fluorescence intensity was 14.8. For CGL27, CGL29, and CGL30, the number of cells was 1 and the fluorescence intensity was 8.9 to 10.3, but these results were concordant or discordant with tissue TPS.

As is apparent from the above results, when the number of PD-L1(+)/CD18(+) cells is 3 or more and the fluorescence intensity is about 19 or more, determining CTC-based cancer immunotherapy may be judged positive. In addition, when the number of PD-L1(+)/CD18(+) cells is 3 or more and the fluorescence intensity is about 15 or less, determining CTC-based cancer immunotherapy may be judged negative.

Consequently, when the number of cells is 1 or less, the method of determining cancer immunotherapy based on CTCs or the concordance with tissue may be regarded as ineffective. Since the number of CTCs that may be regarded as false positives in inflammatory patients is 1 or less, determining cancer immunotherapy with 1 or less CTCs in lung cancer patients may be considered impossible.

Also, referring to fluorescence intensity, since the average fluorescence intensity of CTCs that may be regarded as false positives in inflammatory patients is 11.2, determining cancer immunotherapy may be judged negative when the fluorescence intensity is 11.2 or less. For CGL21, in which both CTC and tissue TPS results are negative in the lung cancer patient group, the number of cells was 3 and the average fluorescence intensity was 14.8, and thus, determining cancer immunotherapy may be judged negative when the fluorescence intensity is 14.8 or less in lung cancer patients.

### [Industrial Applicability]

According to the present invention, a method of treating cancer using circulating tumor cells is capable of selecting a patient by simultaneously confirming expression of an immune checkpoint protein and expression of a lymphoid or myeloid cell-specific protein in liquid biopsy, so that the concordance with a tissue test is very high, resulting in high commercial applicability. Therefore, the method of the present invention is useful for cancer diagnosis and treatment.

## Claims

1. A method of selecting a patient for application of cancer immunotherapy drug, comprising:
(a) confirming expression of an immune checkpoint protein and a lymphoid or myeloid-specific protein in isolated circulating tumor cells; and
(b) classifying a patient in whom immune checkpoint protein expression is confirmed as positive and lymphoid or myeloid-specific protein expression is confirmed as positive as a patient to whom cancer immunotherapy drug is applicable.

2. The method according to claim 1, wherein the immune checkpoint protein is selected from the group consisting of PD-1, PD-L1, and CTLA-4.

3. The method according to claim 1, wherein the lymphoid or myeloid-specific protein is selected from the group consisting of CD18, CD16, CD29, CD61, CD104, CD32, CD11b, and CD64.

4. The method according to claim 1, wherein the circulating tumor cells are isolated by:
(i) obtaining blood from a cancer patient; and
(ii) isolating circulating tumor cells from the blood using a biochip.

5. The method according to claim 1, wherein confirming the expression of the immune checkpoint protein and the lymphoid or myeloid-specific protein is performed by:
(1) carrying out reaction with a fluorescent marker that specifically binds to circulating tumor cells, a fluorescent marker that specifically binds to an immune checkpoint protein, and a fluorescent marker that specifically binds to a lymphoid or myeloid-specific protein;
(2) receiving optical images in multiple wavelength ranges for the circulating tumor cells, the immune checkpoint protein, and the lymphoid or myeloid-specific protein, reacted with the fluorescent markers;
(3) performing primary filtering by measuring fluorescence intensities of the circulating tumor cells, the immune checkpoint protein, and the lymphoid or myeloid-specific protein in optical images of all or part of the multiple wavelength ranges;
(4) performing secondary filtering by measuring morphology of the circulating tumor cells in the optical images of all or part of the multiple wavelength ranges; and
(5) performing tertiary filtering by measuring morphology of the circulating tumor cells in an integrated image obtained by merging all or part of the optical images in the multiple wavelength ranges.

6. The method according to claim 5, wherein the fluorescent marker that specifically binds to the circulating tumor cells is selected from the group consisting of a vimentin-specific antibody, an EpCAM-specific antibody, and a CK-specific antibody.

7. The method according to claim 5, wherein the fluorescent marker that specifically binds to the immune checkpoint protein is selected from the group consisting of a PD-1-specific antibody, a PD-L1-specific antibody, and a CTLA-4-specific antibody.

8. The method according to claim 5, wherein the fluorescent marker that specifically binds to the lymphoid or myeloid protein is selected from the group consisting of a CD18-specific antibody, a CD16-specific antibody, a CD29-specific antibody, a CD61-specific antibody, a CD104-specific antibody, a CD32-specific antibody, a CD11b-specific antibody, and a CD64-specific antibody.

9. The method according to claim 1, wherein the cancer immunotherapy drug is an antibody.

10. The method according to claim 1, wherein the cancer immunotherapy drug is a monoclonal antibody.

11. The method according to claim 1, wherein the cancer immunotherapy drug is a human antibody, a humanized antibody, or a chimeric antibody.

12. The method according to claim 1, wherein the cancer immunotherapy drug is at least one selected from the group consisting of a PD-L1 antagonist, a PD-1 antagonist, and a CTLA-4 antagonist.

13. The method according to claim 1, wherein the cancer immunotherapy drug is at least one selected from the group consisting of pembrolizumab (Keytruda), nivolumab (Opdivo), cemiplimab (Lybtayo), atezolizumab (Tecentriq), avelumab (Bacencio), durvalumab (Imfinzi), ipilimumab (Yervoy), and tremelimumab.

14. The method according to claim 1, wherein the cancer is selected from the group consisting of lung cancer, kidney cancer, bladder cancer, breast cancer, colorectal cancer, ovarian cancer, pancreatic cancer, gastric carcinoma, esophageal cancer, mesothelioma, melanoma, head and neck cancer, thyroid cancer, sarcoma, prostate cancer, glioblastoma, cervical cancer, thymic carcinoma, leukemia, lymphoma, myeloma, mycosis fungoides, Merkel cell cancer, and hematological malignancies,
optionally wherein the cancer is lung cancer, or the cancer is non-small cell lung cancer.

15. An immune checkpoint inhibitor for use in a method of treating cancer, the method comprising:
(a) confirming expression of an immune checkpoint protein and a lymphoid or myeloid-specific protein in circulating tumor cells; and
(b) administering the immune checkpoint inhibitor when immune checkpoint protein expression is positive and lymphoid or myeloid-specific protein expression is positive.

## Patentansprüche

1. Verfahren zum Auswählen eines Patienten zur Anwendung eines Krebs-Immuntherapie-Arzneimittels, das Folgendes umfasst:
(a) das Bestätigen der Expression eines Immun-Checkpoint-Proteins und eines lymphoiden oder myeloid-spezifischen Proteins in isolierten zirkulierenden Tumorzellen; und
(b) das Klassifizieren eines Patienten, bei dem die Expression eines Immun-Checkpoint-Proteins als positiv bestätigt wurde und die Expression eines lymphoiden oder myeloid-spezifischen Proteins als positiv bestätigt wurde, als Patient, bei dem ein Krebs-Immuntherapie-Arzneimittel anwendbar ist.

2. Verfahren nach Anspruch 1, wobei das Immun-Checkpoint-Protein aus der aus PD-1, PD-L1 und CTLA-4 bestehenden Gruppe ausgewählt ist.

3. Verfahren nach Anspruch 1, wobei das lymphoide oder myeloid-spezifische Protein aus der aus CD18, CD16, CD29, CD61, CD104, CD32, CD11b und CD64 bestehenden Gruppe ausgewählt ist.

4. Verfahren nach Anspruch 1, wobei die zirkulierenden Tumorzellen folgendermaßen isoliert werden:
(i) durch Erhalten von Blut von einem Krebspatienten; und
(ii) durch Isolieren von zirkulierenden Tumorzellen aus dem Blut unter Verwendung eines Biochips.

5. Verfahren nach Anspruch 1, wobei das Bestätigen der Expression des Immun-Checkpoint-Proteins und des lymphoiden oder myeloid-spezifischen Proteins folgendermaßen durchgeführt wird:
(1) Durchführung einer Reaktion mit einem Fluoreszenzmarker, der spezifisch an zirkulierende Tumorzellen bindet, einem Fluoreszenzmarker, der spezifisch an ein Immun-Checkpoint-Protein bindet, und einem Fluoreszenzmarker, der spezifisch an ein lymphoides oder myeloid-spezifisches Protein bindet;
(2) Erhalt von optischen Bildern in mehreren Wellenlängen-Bereichen von den zirkulierenden Tumorzellen, dem Immun-Checkpoint-Protein und dem lymphoiden oder myeloid-spezifischen Protein, die mit den Fluoreszenzmarkern umgesetzt wurden:
(3) Durchführung von primärem Filtern durch Messen von Fluoreszenz-Intensitäten der zirkulierenden Tumorzellen, des Immun-Checkpoint-Proteins und des lymphoiden oder myeloid-spezifischen Proteins in optischen Bildern von allen oder einem Teil der mehreren Wellenlängen-Bereiche;
(4) Durchführung von sekundärem Filtern durch Messen der Morphologie der zirkulierenden Tumorzellen in den optischen Bildern von allen oder einem Teil der mehreren Wellenlängen-Bereiche; und
(5) Durchführen von tertiärem Filtern durch Messen der Morphologie der zirkulierenden Tumorzellen in einem integrierten Bild, das durch Zusammenführen von allen oder einem Teil der optischen Bilder in den mehreren Wellenlängen-Bereichen erhalten wurde.

6. Verfahren nach Anspruch 5, wobei der Fluoreszenzmarker, der spezifisch an zirkulierende Tumorzellen bindet, aus der aus einem Vimentin-spezifischen Antikörper, einem EpCAM-spezifischen Antikörper und einem CK-spezifischen Antikörper bestehenden Gruppe ausgewählt ist.

7. Verfahren nach Anspruch 5, wobei der Fluoreszenzmarker, der spezifisch an das Immun-Checkpoint-Protein bindet, aus der aus einem PD-1-spezifischen Antikörper, einem PD-L1-spezifischen Antikörper und einem CTLA-4-spezifischen Antikörper bestehenden Gruppe ausgewählt ist.

8. Verfahren nach Anspruch 5, wobei der Fluoreszenzmarker, der spezifisch an das lymphoide oder myeloide Protein bindet, aus der aus einem CD18-spezifischen Antikörper, einem CD16-spezifischen Antikörper, einem CD29-spezifischen Antikörper, einem CD61-spezifischen Antikörper, einem CD104-spezifischen Antikörper, einem CD32-spezifischen Antikörper, einem CD11b-spezifischen Antikörper und einem CD64-spezifischen Antikörper bestehenden Gruppe ausgewählt ist.

9. Verfahren nach Anspruch 1, wobei das Krebs-Immuntherapie-Arzneimittel ein Antikörper ist.

10. Verfahren nach Anspruch 1, wobei das Krebs-Immuntherapie-Arzneimittel ein monoklonaler Antikörper ist.

11. Verfahren nach Anspruch 1, wobei das Krebs-Immuntherapie-Arzneimittel ein menschlicher Antikörper, ein humanisierter Antikörper oder ein chimärer Antikörper ist.

12. Verfahren nach Anspruch 1, wobei das Krebs-Immuntherapie-Arzneimittel zumindest eines, ausgewählt aus der aus einem PD-L1-Antagonisten, einem PD-1-Antagonisten und einem CTLA-4-Antagonisten bestehenden Gruppe ist.

13. Verfahren nach Anspruch 1, wobei das Krebs-Immuntherapie-Arzneimittel zumindest eines, ausgewählt aus der aus Pembrolizumab (Keytruda), Nivolumab (Opdivo), Cemiplimab (Lybtayo), Atezolizumab (Tecentriq), Avelumab (Bacencio), Durvalumab (Imfinzi), Ipilimumab (Yervoy), und Tremelimumab bestehenden Gruppe ist.

14. Verfahren nach Anspruch 1, wobei der Krebs aus der aus Lungenkrebs, Nierenkrebs, Blasenkrebs, Brustkrebs, Darmkrebs, Eierstockkrebs, Pankreaskrebs, Magenkarzinom, Speiseröhrenkrebs, Mesotheliom, Melanom, Kopf- und Halskrebs, Schilddrüsenkrebs, Sarkom, Prostatakrebs, Glioblastom, Gebärmutterhalskrebs, Thymuskarzinom, Leukämie, Lymphom, Myelom, Mycosis fungoides, Merkelzellkrebs und hämatologischen Tumorerkrankungen bestehenden Gruppe ausgewählt ist,
wobei es sich bei dem Krebs gegebenenfalls um Lungenkrebs handelt oder es sich bei dem Krebs um nicht-kleinzelligen Lungenkrebs handelt.

15. Immun-Checkpoint-Hemmer zur Verwendung in einem Verfahren zur Krebsbehandlung, wobei das Verfahren Folgendes umfasst:
(a) das Bestätigen der Expression eines Immun-Checkpoint-Proteins und eines lymphoiden oder myeloid-spezifischen Proteins in zirkulierenden Tumorzellen; und
(b) das Verabreichen des Immun-Checkpoint-Hemmers, wenn die Expression von Immun-Checkpoint-Protein positiv ist und die Expression von lymphoidem oder myeloidspezifischem Protein positiv ist.

## Revendications

1. Procédé de sélection d'un patient pour l'administration d'un médicament d'immunothérapie anticancéreuse, comprenant les étapes consistant à :
(a) confirmer l'expression d'une protéine point de contrôle immunitaire et d'une protéine spécifique de la lignée lymphoïde ou myéloïde dans des cellules tumorales circulantes isolées ; et
(b) classer un patient chez lequel l'expression de la protéine point de contrôle immunitaire est confirmée positive et l'expression de la protéine spécifique de la lignée lymphoïde ou myéloïde est confirmée positive comme patient auquel le médicament d'immunothérapie anticancéreuse est applicable.

2. Procédé selon la revendication 1, dans lequel la protéine point de contrôle immunitaire est choisie dans le groupe constitué par PD-1, PD-L1 et CTLA-4.

3. Procédé selon la revendication 1, dans lequel la protéine spécifique de la lignée lymphoïde ou myéloïde est choisie dans le groupe constitué par CD18, CD16, CD29, CD61, CD104, CD32, CD11b et CD64.

4. Procédé selon la revendication 1, dans lequel les cellules tumorales circulantes sont isolées par :
(i) prélèvement de sang chez un patient atteint de cancer ; et
(ii) isolement des cellules tumorales circulantes à partir du sang au moyen d'une biopuce.

5. Procédé selon la revendication 1, dans lequel la confirmation de l'expression de la protéine point de contrôle immunitaire et de la protéine spécifique de la lignée lymphoïde ou myéloïde est réalisée par :
(1) réalisation d'une réaction avec un marqueur fluorescent qui se lie spécifiquement aux cellules tumorales circulantes, un marqueur fluorescent qui se lie spécifiquement à une protéine point de contrôle immunitaire, et un marqueur fluorescent qui se lie spécifiquement à une protéine spécifique de la lignée lymphoïde ou myéloïde ;
(2) acquisition d'images optiques dans plusieurs plages de longueurs d'onde pour les cellules tumorales circulantes, la protéine point de contrôle immunitaire et la protéine spécifique de la lignée lymphoïde ou myéloïde ayant réagi avec les marqueurs fluorescents ;
(3) réalisation d'un filtrage primaire par mesure des intensités de fluorescence des cellules tumorales circulantes, de la protéine point de contrôle immunitaire et de la protéine spécifique de la lignée lymphoïde ou myéloïde dans des images optiques de tout ou partie des plages de longueurs d'onde multiples ;
(4) réalisation d'un filtrage secondaire par mesure de la morphologie des cellules tumorales circulantes dans les images optiques de tout ou partie des plages de longueurs d'onde multiples ; et
(5) réalisation d'un filtrage tertiaire par mesure de la morphologie des cellules tumorales circulantes dans une image intégrée obtenue en fusionnant tout ou partie des images optiques dans les plages de longueurs d'onde multiples.

6. Procédé selon la revendication 5, dans lequel le marqueur fluorescent qui se lie spécifiquement aux cellules tumorales circulantes est choisi dans le groupe constitué par un anticorps spécifique de la vimentine, un anticorps spécifique d'EpC1VI et un anticorps spécifique de CK.

7. Procédé selon la revendication 5, dans lequel le marqueur fluorescent qui se lie spécifiquement à la protéine point de contrôle immunitaire est choisi dans le groupe constitué par un anticorps spécifique de PD-1, un anticorps spécifique de PD-L1 et un anticorps spécifique de CTLA-4.

8. Procédé selon la revendication 5, dans lequel le marqueur fluorescent qui se lie spécifiquement à la protéine lymphoïde ou myéloïde est choisi dans le groupe constitué par un anticorps spécifique de CD18, un anticorps spécifique de CD16, un anticorps spécifique de CD29, un anticorps spécifique de CD61, un anticorps spécifique de CD104, un anticorps spécifique de CD32, un anticorps spécifique de CD11b et un anticorps spécifique de CD64.

9. Procédé selon la revendication 1, dans lequel le médicament d'immunothérapie anticancéreuse est un anticorps.

10. Procédé selon la revendication 1, dans lequel le médicament d'immunothérapie anticancéreuse est un anticorps monoclonal.

11. Procédé selon la revendication 1, dans lequel le médicament d'immunothérapie anticancéreuse est un anticorps humain, un anticorps humanisé ou un anticorps chimérique.

12. Procédé selon la revendication 1, dans lequel le médicament d'immunothérapie anticancéreuse est au moins un élément choisi dans le groupe constitué par un antagoniste de PD-L1, un antagoniste de PD-1 et un antagoniste de CTLA-4.

13. Procédé selon la revendication 1, dans lequel le médicament d'immunothérapie anticancéreuse est au moins un élément choisi dans le groupe constitué par le pembrolizumab (Keytruda), le nivolumab (Opdivo), le cémiplimab (Lybtayo), l'atézolizumab (Tecentriq), l'avélumab (Bacencio), le durvalumab (Imfinzi), l'ipilimumab (Yervoy) et le trémélimumab.

14. Procédé selon la revendication 1, dans lequel le cancer est choisi dans le groupe constitué par le cancer du poumon, le cancer du rein, le cancer de la vessie, le cancer du sein, le cancer colorectal, le cancer de l'ovaire, le cancer du pancréas, le carcinome gastrique, le cancer de l'œsophage, le mésothéliome, le mélanome, le cancer de la tête et du cou, le cancer de la thyroïde, le sarcome, le cancer de la prostate, le glioblastome, le cancer du col de l'utérus, le carcinome thymique, la leucémie, le lymphome, le myélome, le mycosis fongoïde, le cancer à cellules de Merkel et des affections hématologiques malignes,
le cas échéant, dans lequel le cancer est un cancer du poumon, ou le cancer est un cancer du poumon non à petites cellules.

15. Inhibiteur de point de contrôle immunitaire destiné à être utilisé dans un procédé de traitement du cancer, le procédé comprenant les étapes consistant à :
(a) confirmer l'expression d'une protéine point de contrôle immunitaire et d'une protéine spécifique de la lignée lymphoïde ou myéloïde dans des cellules tumorales circulantes ; et
(b) administrer l'inhibiteur de point de contrôle immunitaire lorsque l'expression de la protéine point de contrôle immunitaire est positive et l'expression de la protéine spécifique de la lignée lymphoïde ou myéloïde est positive.
